# FASCICULE DE BREVET EUROPEEN

(11) **EP 3 621 504 B1**
(45) Date de publication et mention de la délivrance du brevet: **09.11.2022**
(21) Numéro de dépôt: 18728439.3
(22) Date de dépôt: 09.05.2018
(51) Int. Cl.: A47K 7/04, A61L 2/22, A61B 90/80

(54) **APPAREIL DE DESINFECTION PAR NEBULISATION**
DESINFEKTIONSVORRICHTUNG UNTER VERWENDUNG VON VERNEBELUNG
DISINFECTION APPLIANCE USING NEBULISATION

(30) Priorité: 11.05.2017 FR 1754122
(43) Date de publication de la demande: 18.03.2020
(73) Titulaire: Evercleanhand, 38610 Venon (FR)
(72) Inventeur: KECHICHIAN, Asbed, 38610 Venon (FR); BELLE, Guillaume, 38660 Lumbin (FR)
(74) Mandataire: INNOV-GROUP
(86) Numéro de dépôt international: PCT/FR2018/051156
(87) Numéro de publication internationale: WO 2018/206899

(56) Documents cités:
- US-A- 3 918 987
- US-A1- 2013 283 629

## Description

### DOMAINE TECHNIQUE

Le domaine de l'invention est celui des appareils de désinfection par nébulisation d'une solution désinfectante destinée à se déposer sur la surface d'un élément placé dans une enceinte de désinfection.

### ÉTAT DE LA TECHNIQUE ANTÉRIEURE

Dans un certain nombre de milieux, il importe que les personnes puissent se désinfecter les mains dans le but d'éviter la propagation des infections. C'est par exemple le cas, entre autres, du milieu médical pour le personnel soignant, les visiteurs et les patients, le milieu de la restauration et de l'agroalimentaire pour les opérateurs, le personnel de cuisine et les clients, ainsi que le milieu des structures d'accueil infantile et de gériatrie...

Pour cela, des appareils de désinfection existent, qui comportent une enceinte de désinfection destinée à recevoir la ou les mains à désinfecter d'un l'utilisateur, et un dispositif adapté à pulvériser ou nébuliser une solution désinfectante dans l'enceinte de désinfection. Le document US 2013/283629 A1 divulgue un tel appareil de désinfection.

A titre d'exemple, le document WO2011/086323 décrit un appareil de désinfection par nébulisation d'une solution désinfectante. L'appareil de désinfection comporte ainsi une enceinte au sein de laquelle est disposée une pluralité d'injecteurs adaptés à vaporiser la solution désinfectante sous la forme d'un spray divergent, en direction de la main introduite. Les injecteurs sont placés notamment de manière à permettre la diffusion de la solution désinfectante depuis l'extrémité des doigts vers le poignet. L'appareil de désinfection comporte également un dispositif pour générer un flux d'air horizontal dans l'enceinte de désinfection. Il comporte enfin des écarteurs disposés dans l'enceinte de désinfection, ces écarteurs se présentant sous la forme de tiges verticales contre lesquelles la main vient en butée, obligeant ainsi l'utilisateur à écarter les doigts de la main.

Cependant, la présence de ces écarteurs peut perturber l'écoulement de l'aérosol désinfectant dans l'enceinte de désinfection, et ainsi réduire la répartition de la solution désinfectante sur la surface de la main. De plus, il peut être recherché d'éviter que les mains des utilisateurs successifs puissent toucher un tel écarteur. Enfin, il peut également être recherché de permettre à l'utilisateur de bouger sa main dans l'enceinte de désinfection pour homogénéiser le dépôt de l'aérosol sur la surface de la main.

### EXPOSÉ DE L'INVENTION

L'invention a pour objectif de proposer un appareil de désinfection par nébulisation présentant un taux élevé de recouvrement de l'élément à désinfecter par un produit désinfectant nébulisé, conduisant ainsi à un taux de désinfection important, sans avoir recours à la présence d'un organe fixe présent dans l'enceinte de désinfection contre lequel l'élément à désinfecter peut venir en contact.

Pour cela, l'objet de l'invention est un appareil de désinfection selon la revendication 1 comportant :
- une enceinte de désinfection, délimitée par une paroi comportant une ouverture d'entrée par laquelle un élément à désinfecter peut être introduit, et une ouverture de sortie opposée à l'ouverture d'entrée suivant un axe longitudinal suivant lequel s'étend longitudinalement la paroi ;
- un dispositif de nébulisation, adapté à nébuliser un produit désinfectant sous forme d'aérosol ;
- un dispositif d'injection, comportant au moins un injecteur d'aérosol, adapté à injecter dans l'enceinte de désinfection l'aérosol formé par le dispositif de nébulisation.

Selon l'invention, le dispositif d'injection comporte en outre au moins un injecteur d'air, adapté à injecter de l'air au travers d'un orifice d'injection de la paroi, suivant une direction d'injection, et à générer une convection forcée dans l'enceinte de désinfection et rotative autour de l'axe longitudinal, l'injecteur d'air étant agencé de sorte que la direction d'injection est, en projection dans un plan perpendiculaire à l'axe longitudinal, inclinée vis-à-vis d'un axe dit de position, ledit axe de position étant perpendiculaire à l'axe longitudinal, et passant par ledit orifice d'injection dudit injecteur d'air.

Ainsi, l'axe de position est sécant de l'axe longitudinal et orthogonal à celui-ci, et passe par l'orifice d'injection d'air. Par cet agencement de l'injecteur d'air vis-à-vis de l'enceinte de désinfection, une convection forcée d'air et donc d'aérosol est générée dans l'enceinte, qui est rotative autour de l'axe longitudinal. Par inclinée, on entend que la direction d'injection forme un angle d'inclinaison non nul par rapport à l'axe de position, en projection dans un plan perpendiculaire à l'axe longitudinal. Ainsi, que l'élément à désinfecter soit introduit ou non dans l'enceinte de désinfection, la convection d'air et donc d'aérosol est rotative autour de l'axe longitudinal. Par convection rotative autour d'un axe, on entend ici que l'écoulement d'air et d'aérosol présente un mouvement principal de rotation autour de l'axe considéré. Il est également qualifié d'écoulement tourbillonnaire. Ainsi, une particule fluide présente, dans l'enceinte de désinfection, un mouvement de rotation autour de l'axe longitudinal.

Le dispositif d'injection comporte en outre un moyen de convection forcée, agencé vis-à-vis de l'enceinte de désinfection de manière à générer un écoulement d'air allant de l'ouverture d'entrée à l'ouverture de sortie.

Certains aspects préférés, mais non limitatifs de cet appareil de désinfection sont les suivants.

Le dispositif d'injection peut comporter au moins un injecteur d'air, et au moins un injecteur d'aérosol distinct de l'injecteur d'air.

Le dispositif d'injection peut comporter au moins un injecteur d'air et d'aérosol adapté à former ledit injecteur d'air et ledit injecteur d'aérosol.

L'appareil de désinfection peut comporter une pluralité d'injecteurs d'air présentant chacun, en projection dans le plan perpendiculaire à l'axe longitudinal, un angle d'inclinaison non nul formé par sa direction d'injection vis-à-vis de son axe de position, les angles d'inclinaison étant de même signe.

Un filtre apte à collecter l'aérosol peut être situé en regard de l'ouverture de sortie de l'enceinte de désinfection, c'est-à-dire en face de l'ouverture. Ainsi, l'aérosol sortant par l'ouverture de sortie est collecté par le filtre.

L'enceinte de désinfection peut être agencée de sorte que l'axe longitudinal est incliné vis-à-vis de l'axe de la gravité, l'ouverture de sortie étant située au-dessus de l'ouverture d'entrée. En d'autres termes, l'ouverture de sortie est destinée à être située au-dessus de l'ouverture d'entrée suivant l'axe de gravité.

L'injecteur d'air peut comporter un ventilateur.

Le dispositif de nébulisation peut comporter une chambre de nébulisation comportant une solution liquide désinfectante, dans laquelle est situé un nébuliseur adapté à transformer la solution liquide désinfectante en aérosol, l'injecteur d'aérosol comportant au moins un conduit d'injection reliant fluidiquement la chambre de nébulisation à l'enceinte de désinfection.

Le dispositif de nébulisation peut comporter une chambre de nébulisation comportant une solution liquide désinfectante, dans laquelle est situé un nébuliseur adapté à transformer la solution liquide désinfectante en aérosol, la chambre de nébulisation et l'enceinte de désinfection étant chacune délimitée au moins en partie par une même paroi comportant au moins un orifice d'injection traversant autorisant le passage de l'aérosol de la chambre de nébulisation dans l'enceinte de désinfection.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
les figures 1 et 2 sont des vues schématiques, en coupe suivant l'axe longitudinal Δ, respectivement en deux dimensions et en perspective, de l'appareil de désinfection selon le premier mode de réalisation ;
les figures 3A à 3C sont des vues en coupe transversale de l'enceinte de désinfection pour différentes variantes de l'enceinte de désinfection et du dispositif d'injection de l'appareil de désinfection selon le premier mode de réalisation ;
la figure 4 est une vue schématique, en coupe suivant l'axe longitudinal Δ et en perspective, d'un appareil de désinfection selon une autre variante du premier mode de réalisation ;
la figure 5 est une vue schématique, en coupe suivant l'axe longitudinal Δ et en perspective, d'un appareil de désinfection selon une autre variante du premier mode de réalisation ;
la figure 6 est une vue schématique, en coupe suivant l'axe longitudinal Δ et en perspective, d'un appareil de désinfection selon un deuxième mode de réalisation ;
les figures 7A à 7C sont des vues en coupe transversale de l'enceinte de désinfection pour différentes variantes de l'enceinte de désinfection et du dispositif d'injection de l'appareil de désinfection selon le deuxième mode de réalisation.

### EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près. Par ailleurs, l'expression « comportant un » doit être comprise comme « comportant au moins un », sauf indication contraire.

L'invention porte sur un appareil de désinfection par nébulisation. L'appareil de nébulisation comporte une enceinte de désinfection adaptée à recevoir un élément à désinfecter, un dispositif de nébulisation adapté à générer un aérosol d'une solution désinfectante destinée à se déposer sur l'élément introduit dans l'enceinte, et un dispositif d'injection adapté à injecter l'aérosol généré dans l'enceinte de désinfection.

Par nébulisation, on entend la transformation en un aérosol d'un liquide initialement en phase continue. L'aérosol est une suspension ou un nuage de fines gouttelettes de liquide. La taille moyenne des gouttelettes peut être de l'ordre de quelques microns à quelques dizaines de microns, et est avantageusement inférieure à 30µm.

L'enceinte de désinfection est délimitée par une paroi comportant une ouverture d'entrée par laquelle l'élément à désinfecter peut être introduit, et une ouverture de sortie opposée à l'ouverture d'entrée suivant un axe longitudinal Δ suivant lequel s'étend longitudinalement la paroi. L'entrée et la sortie sont des ouvertures pratiquées dans la paroi délimitant l'enceinte de désinfection. L'axe longitudinal Δ est défini comme étant un axe rectiligne allant de l'entrée à la sortie et passant par les barycentres des sections droites de l'enceinte de désinfection.

Le dispositif d'injection comporte au moins un injecteur d'aérosol adapté à injecter dans l'enceinte de désinfection l'aérosol formé par le dispositif de nébulisation. Il comporte en outre au moins un injecteur d'air, adapté à injecter de l'air au travers d'un orifice d'injection de la paroi suivant une direction d'injection, et à générer une convection forcée dans l'enceinte de désinfection qui soit rotative, ou tournante, autour de l'axe longitudinal Δ. Pour cela, l'injecteur d'air est agencé vis-à-vis de la paroi de l'enceinte de désinfection de sorte que la direction d'injection, en projection dans un plan perpendiculaire à l'axe longitudinal Δ, est inclinée par rapport à un axe dit de position, l'axe de position étant orthogonal à l'axe longitudinal Δ, et passant par cet axe longitudinal Δ et l'orifice d'injection de l'injecteur d'air considéré. La direction d'injection d'air est dite inclinée dans le sens où elle forme un angle d'inclinaison non nul par rapport à cet axe de position. L'angle d'inclinaison est ainsi supérieur strictement à 0°, et est de préférence supérieur à 10° voire à 20°, ou davantage, par exemple égal à 30°, à 45°.... Comme détaillé plus loin, l'injecteur d'air et l'injecteur d'aérosol peuvent être distincts l'un de l'autre, ou confondus l'un avec l'autre.

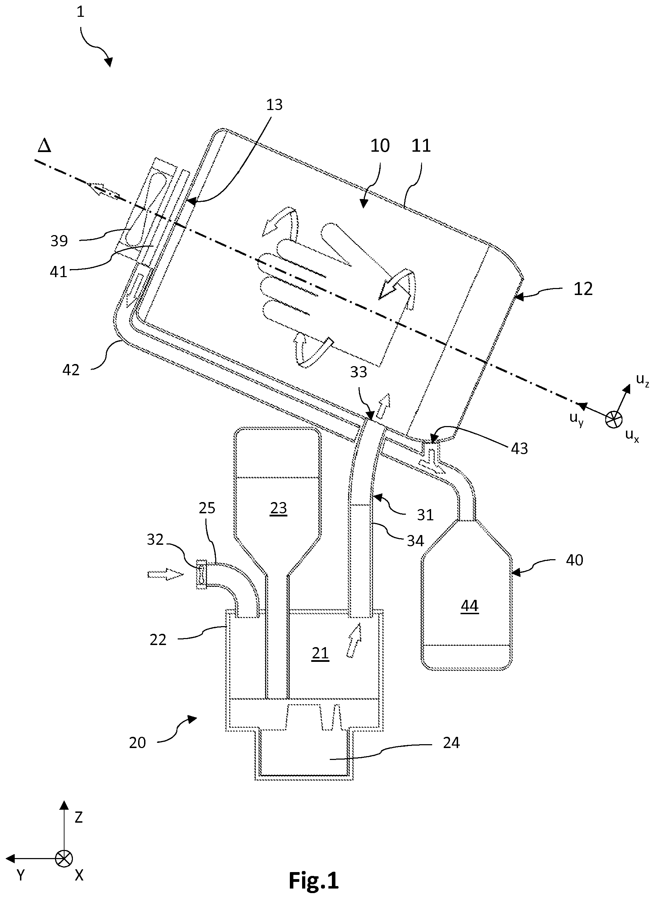

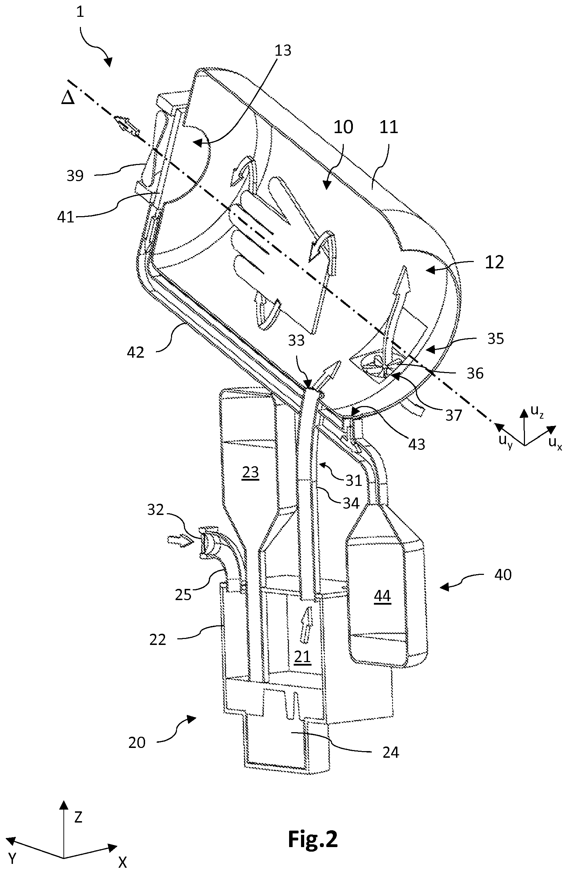

Les figures 1 et 2 sont des vues schématiques, respectivement bidimensionnelle et en perspective, de l'appareil de désinfection 1 selon un premier mode de réalisation, celui-ci étant représenté selon un plan de coupe longitudinale passant par l'axe longitudinal Δ.

On définit ici et pour la suite de la description un repère direct tridimensionnel orthogonal (X,Y,Z), où l'axe Y est orienté de manière parallèle à l'axe longitudinal Δ et où l'axe Z est orienté suivant l'axe de la gravité. Dans la suite de la description, les termes « vertical » et « verticalement » s'entendent comme étant relatifs à une orientation sensiblement parallèle à l'axe Z, et les termes « horizontal » et « horizontalement » comme étant relatifs à une orientation sensiblement parallèle au plan (X,Y). Par ailleurs, les termes « inférieur » et « supérieur » s'entendent comme étant relatifs à un positionnement croissant suivant la direction +Z. On définit également un repère (uₓ, u_{y}, u_{z}) en coordonnées cylindriques, où le vecteur u_{y} est colinéaire à l'axe longitudinal Δ et orienté vers la sortie 13.

L'appareil de désinfection 1 comporte l'enceinte de désinfection 10 adaptée à recevoir un élément à désinfecter, ici une main d'un utilisateur, un dispositif de nébulisation 20 adapté à générer l'aérosol désinfectant destiné à se déposer sur la main présente dans l'enceinte de désinfection 10, et un dispositif d'injection 30 adapté à injecter dans l'enceinte de désinfection 10 l'aérosol ainsi formé. Il comporte avantageusement un dispositif de collecte 40 adapté à collecter l'aérosol désinfectant ou le liquide désinfectant présent dans l'enceinte de désinfection 10, mais n'ayant pas été utilisé.

L'enceinte de désinfection 10 est délimitée spatialement par une paroi 11, et s'étend longitudinalement suivant l'axe Δ. Celle-ci comporte une ouverture d'entrée 12 par laquelle la main de l'utilisateur est introduite dans l'enceinte de désinfection 10, et une ouverture de sortie 13 opposée à l'ouverture d'entrée 12 suivant l'axe longitudinal Δ. Elle présente une section droite, orthogonale à l'axe longitudinal Δ, de forme circulaire. Elle peut en variante présenter d'autres formes, par exemple ovale, voire polygonale, tel que carrée, rectangulaire ou autre.

L'enceinte de désinfection 10 peut présenter une orientation inclinée vis-à-vis de l'axe de la gravité, de telle sorte que l'entrée 12 est agencée plus bas que la sortie 13 suivant l'axe Z. Dans cet exemple, comme décrit plus loin, la paroi 11 peut alors comporter un orifice de collecte 43 relié fluidiquement à un réservoir 44 d'un dispositif de collecte 40. L'orifice de collecte 43 est adapté à collecter l'aérosol s'étant éventuellement déposé sur la surface interne de la paroi 11 et ayant coalescé sous forme de gouttes. L'orifice de collecte 43 est alors placé dans une zone dite inférieure de la paroi 11 suivant l'axe Z, sous l'entrée 12, de sorte que de telles gouttes peuvent ainsi s'écouler sous l'effet de la gravité jusqu'à rejoindre l'orifice de collecte 43.

L'enceinte de désinfection 10 comporte en outre au moins un orifice d'injection 37 d'air par lequel de l'air est injecté dans l'enceinte 10 de manière à y générer un écoulement d'air rotatif autour de l'axe longitudinal Δ, et au moins un orifice d'injection 33 d'aérosol par lequel l'aérosol généré est introduit dans l'enceinte 10. L'orifice d'injection 37 d'air et l'orifice d'injection 33 d'aérosol sont ici distincts l'un de l'autre.

Le dispositif de nébulisation 20 est adapté à transformer en aérosol une solution se présentant initialement en phase liquide continue, l'aérosol étant ensuite destiné à être introduit dans l'enceinte de désinfection 10. Il comporte dans cet exemple une chambre de nébulisation 21, un réservoir 23 de produit désinfectant, et un nébuliseur 24.

La chambre de nébulisation 21 est délimitée par une paroi 22 et est partiellement remplie par le liquide désinfectant. Elle est reliée fluidiquement au réservoir 23 par un conduit, le réservoir 23 permettant de maintenir constante la hauteur de liquide désinfectant dans la chambre de nébulisation 21.

Le liquide désinfectant peut être, à titre d'exemples, une solution hydro-alcoolique, une solution à base d'hypochlorite de sodium ou de povidone iodée, une solution à base de chlorure d'ammonium et d'amine oxyde, de l'eau ozonée, ou toute autre solution désinfectante pouvant être nébulisée.

Dans cet exemple, le nébuliseur 24 est type piézoélectrique à membrane vibrante à ultrasons. Il comporte ainsi une membrane à mettre en vibration et un élément piézoélectrique (non représentés), par exemple une céramique, permettant de mettre en vibration la membrane en transformant une tension d'excitation en vibration. La membrane vibrante est ainsi liée à la céramique, elle-même étant assemblée à un boîtier. Le boîtier du nébuliseur 24 est placé dans la chambre de nébulisation 21 de sorte que la surface du liquide recouvre la membrane vibrante. La vibration de la membrane par effet piézoélectrique provoque la formation de l'aérosol par transmission d'ultrasons, l'aérosol formé étant alors composé de gouttelettes du liquide désinfectant. Les gouttelettes ont une taille moyenne avantageusement inférieure à quelques dizaines de microns, par exemple 30µm, et de préférence inférieure ou égale à 15µm. Par exemple, la taille moyenne des gouttelettes peut être de l'ordre de 5µm à 10µm environ. Un tel nébuliseur 24 est connu de l'homme du métier et n'est pas décrit plus en détail.

D'autres types de nébuliseurs peuvent, en variante, être utilisés, par exemple des nébuliseurs à effet Venturi, ou des nébuliseurs piézoélectriques à membrane perforée vibrante au travers de laquelle un liquide s'écoule et est transformé en aérosol par la vibration de la membrane. Un exemple d'un tel nébuliseur est notamment décrit dans le document FR2886174.

Le dispositif d'injection 30 est adapté, d'une part, à introduire dans l'enceinte de désinfection 10 l'aérosol formé par le dispositif de nébulisation 20, et d'autre part, à générer par convection forcée un flux d'air, entraînant alors l'aérosol présent, de manière rotative autour de l'axe longitudinal Δ. Le dispositif d'injection 30 comporte un ou plusieurs injecteurs d'air 35, et un ou plusieurs injecteurs d'aérosol 31 distincts des injecteurs d'air 35. Il comporte un injecteur d'air 35 et un injecteur d'aérosol 31.

L'injecteur d'air 35 et l'injecteur d'aérosol 31 comportent chacun des moyens de convection forcée adaptés à générer un écoulement d'air et/ou d'aérosol au travers d'un ou plusieurs orifices d'injection 33, 37 de la paroi 11 de l'enceinte de désinfection 10. Les moyens de convection forcée peuvent être motorisés, et comporter une partie mobile, telle que des pâles ou une hélice, entraînée en rotation. Il s'agit avantageusement d'un ventilateur, dont l'axe du flux d'air forcé (et donc de la direction d'injection) est défini par l'orientation du ventilateur et/ou par la présence d'un canalisateur d'air (non représenté) situé en aval du ventilateur. Le canalisateur d'air peut être une ailette agencée de manière à orienter le flux d'air forcé suivant une direction d'injection déterminée. Le ventilateur peut présenter une puissance électrique de quelques watts, par exemple de l'ordre de 1 Watt, et provoquer un débit d'air forcé. Ainsi, il présente une faible consommation d'énergie tout en offrant une bonne efficacité d'injection d'air. Il peut présenter un encombrement limité et un faible bruit de fonctionnement. Ainsi, l'injecteur d'air peut comporter un ventilateur, ou équivalent, disposé au niveau de l'orifice d'injection 37, dont l'axe de flux d'air est orienté de sorte que la direction d'injection D, en projection dans un plan perpendiculaire à l'axe Δ, est inclinée vis-à-vis de l'axe de position OP, de préférence d'un angle d'inclinaison supérieur ou égal à 10°, voire à 20°, par exemple à 30° ou à 40°, voire davantage. En variante, le ventilateur peut être présent en amont de l'orifice d'injection 37, auquel cas le conduit d'injection 34 est orienté et débouche au niveau de l'orifice d'injection 37 de sorte que la direction d'injection D est inclinée comme précisé précédemment. Quoi qu'il en soit, la direction d'injection D peut également être définie par un canalisateur d'air situé au niveau de l'orifice d'injection 37.

L'injecteur d'aérosol 31 comporte un moyen de convection forcée 32 et un conduit d'injection 34 reliant fluidiquement la chambre de nébulisation 21 à un orifice d'injection 33 d'aérosol. Le moyen de convection forcée est ici un ventilateur 32 agencé vis-à-vis de la chambre de nébulisation 21 de manière à y insuffler de l'air, et donc à y induire une surpression. Le ventilateur 32 est ici situé dans un conduit d'entrée 25 communiquant avec un orifice situé dans une partie haute de la paroi 22 de la chambre de nébulisation 21. Le flux d'air induit par le ventilateur 32 provoque un écoulement de l'aérosol généré, à partir de la chambre de nébulisation 21, dans le conduit d'injection 34, jusqu'à l'orifice d'injection 33, de manière à être introduit dans l'enceinte de désinfection 10.

Le conduit d'injection 34 d'aérosol ne présente avantageusement pas de pertes de charge singulières, induites par exemple par un changement brusque d'orientation, susceptible de provoquer une coalescence locale d'une partie de l'aérosol. Pour cela, la chambre de nébulisation 21 et l'orifice d'injection 33 sont agencés mutuellement de sorte que le conduit d'injection 34 est sensiblement rectiligne.

L'injecteur d'air 35 (cf. figure 2) comporte un moyen de convection forcée 36, ici un ventilateur, assemblé à la paroi 11 de l'enceinte de désinfection 10 et placé dans, ou au niveau de l'orifice d'injection 37 d'air.

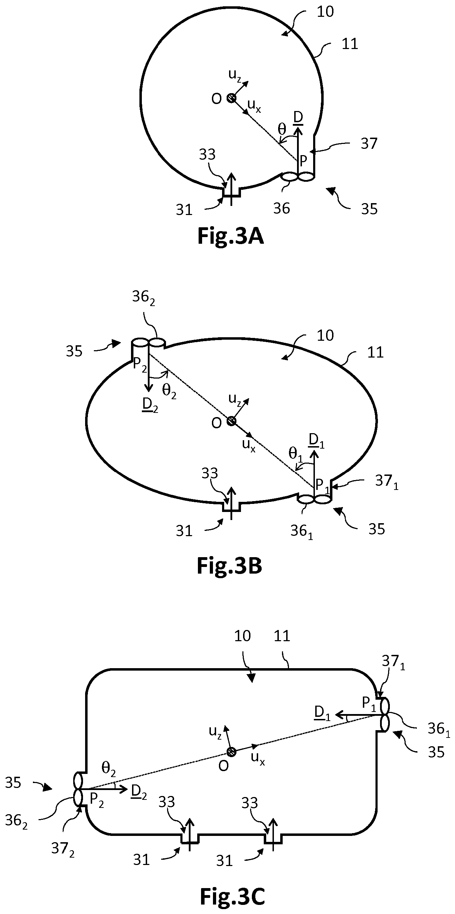

Comme l'illustre schématiquement la figure 3A, le ventilateur 36 présente une direction D d'injection d'air inclinée, en projection dans un plan perpendiculaire à l'axe longitudinal Δ, vis-à-vis de l'axe de position OP correspondant, ledit axe de position étant orthogonal à l'axe longitudinal Δ, et passant par ledit axe longitudinal Δ et l'orifice d'injection 37 d'air. Autrement dit, la direction d'injection D forme, en projection dans le plan perpendiculaire à l'axe Δ, un angle d'inclinaison non nul vis-à-vis de l'axe de position OP. L'angle d'inclinaison est ici défini comme θᵢ=(Dᵢ,PᵢO), où Dᵢ est un vecteur orienté suivant la direction d'injection et projeté dans le plan perpendiculaire à l'axe Δ, et PiO est un vecteur allant de la position Pi de l'orifice d'injection d'air considéré à la position O correspondant à l'intersection entre l'axe de position et l'axe longitudinal Δ, également en projection dans le plan perpendiculaire à l'axe Δ,. Par convention, le signe de l'angle d'inclinaison θᵢ est défini selon le sens trigonométrique dans un plan (uₓ,u_{z}) d'un repère (uₓ,u_{y},u_{z}) en coordonnées cylindriques où le vecteur u_{y} est colinéaire à l'axe longitudinal Δ et orienté vers la sortie 13. Le plan (uₓ,u_{z}) est ainsi un plan perpendiculaire à l'axe longitudinal Δ. L'angle d'inclinaison est strictement supérieur à 0°, et est de préférence supérieur à 10° voire à 20°, par exemple sensiblement égal à 30°, voire davantage.

Les figures 3B et 3C illustrent des variantes de l'appareil de désinfection 1 selon le premier mode de réalisation, qui se distinguent de l'appareil représenté sur la figure 3A notamment en ce que le dispositif d'injection 30 comporte plusieurs injecteurs d'air 35. Dans ce cas, les angles d'inclinaison des injecteurs d'air 35 présentent avantageusement le même signe, dans le plan (uₓ,u_{z}) perpendiculaire à l'axe longitudinal Δ, de manière à renforcer le caractère rotatif du flux d'air, entraînant alors l'aérosol, autour de l'axe longitudinal Δ.

La figure 3B illustre schématiquement une enceinte de désinfection 10 de section droite ovale, dans un plan de coupe parallèle au plan (uₓ,u_{z}). Le dispositif d'injection 30 comporte un seul injecteur d'aérosol 31 et deux injecteurs d'air 35 à ventilateurs. Un premier ventilateur 36₁ présente une direction d'injection D₁ qui, en projection dans un plan perpendiculaire à l'axe longitudinal Δ, forme un angle d'inclinaison θ₁ par rapport à l'axe de position porté par le vecteur P₁O, ici de l'ordre de +60°. Le deuxième ventilateur 36₂ présente une direction d'injection D₂ qui, en projection dans un plan perpendiculaire à l'axe longitudinal Δ, forme un angle d'inclinaison θ₂ par rapport à l'axe de position correspondant porté par le vecteur P₂O, ici également de l'ordre de +60°. Les angles d'inclinaison θ₁, θ₂ peuvent être de valeurs différentes, mais sont de même signe, de sorte que la convection forcée dans l'enceinte de désinfection 10 est bien rotative autour de l'axe longitudinal Δ.

La figure 3C illustre schématiquement une enceinte de désinfection 10 de section droite rectangulaire à coins arrondis, dans le plan de coupe (uₓ,u_{z}) orthogonal à l'axe longitudinal Δ. Le dispositif d'injection 30 comporte deux injecteurs d'aérosol 31 et deux injecteurs d'air 35 comportant des ventilateurs. Un premier ventilateur 36₁ présente une direction d'injection D₁ qui forme, en projection dans un plan perpendiculaire à l'axe longitudinal Δ, un angle d'inclinaison θ₁ par rapport à l'axe de position porté par le vecteur P₁O, ici de l'ordre de +30°. Le deuxième ventilateur 36₂ présente une direction d'injection D₂ qui forme, en projection dans un plan perpendiculaire à l'axe longitudinal Δ, un angle d'inclinaison θ₂ par rapport à l'axe de position correspondant porté par le vecteur P₂O, ici également de l'ordre de +30°. Les angles d'inclinaison θ₁, θ₂ étant de même signe, le flux d'air forcé dans l'enceinte de désinfection 10 est bien rotatif autour de l'axe longitudinal Δ.

De préférence, le dispositif d'injection 30 comporte des moyens supplémentaires 39 de convection forcée adaptés à induire dans l'enceinte de désinfection 10 un écoulement d'air allant de l'entrée 12 vers la sortie 13. Ces moyens de convection forcée 39 peuvent être un ou plusieurs ventilateurs, placés à l'entrée 12 et/ou à la sortie 13 de l'enceinte de désinfection 10. Dans cet exemple, un ventilateur 39 est assemblé à la paroi 11 de l'enceinte de désinfection 10 au niveau de la sortie 13, et ici placé à l'extérieur de l'enceinte 10. Il permet d'aspirer l'air présent dans l'enceinte de désinfection 10 vers la sortie 13. Ainsi, outre l'écoulement rotatif de l'aérosol autour de l'axe longitudinal Δ, l'aérosol s'écoule également de manière dirigée vers la sortie 13, ce qui permet de limiter les risques de perte de l'aérosol par diffusion de celui-ci hors de l'enceinte de désinfection 10 par l'ouverture d'entrée 12, tout en améliorant l'homogénéité du dépôt de l'aérosol sur la surface de la main.

De préférence, l'appareil de désinfection 1 comporte un dispositif de collecte 40 adapté à récupérer le liquide désinfectant ou l'aérosol non déposé sur la main de l'utilisateur. Le dispositif de collecte 40 comporte dans cet exemple un filtre de condensation 41 placé à la sortie 13 et à l'extérieur de l'enceinte de désinfection 10, ici entre la paroi 11 et le ventilateur 39 de sortie. Un conduit 42 relie fluidiquement le filtre de condensation 41 à un réservoir de collecte 44. Ainsi, l'aérosol non déposé sur la main s'écoule jusqu'à la sortie 13 et est capté par le filtre 41. Les microgouttelettes coalescent dans le filtre 41, et le liquide formé s'écoule par gravité dans le conduit 42 jusqu'au réservoir 44.

Le dispositif de collecte 40 comporte en outre au moins un orifice de collecte 43 traversant la paroi 11, et placé dans une zone inférieure de l'enceinte de désinfection 10, sous l'entrée 12. Ainsi, dans l'éventualité où une partie des gouttelettes de l'aérosol se déposent sur la surface interne de la paroi 11 et coalescent les unes avec les autres, le liquide formé peut s'écouler par gravité dans l'enceinte de désinfection 10, et être collecté par l'orifice de collecte 43. Le liquide s'écoule alors dans le conduit 42 jusqu'au réservoir 44. Le liquide collecté peut alors subir un traitement dans le but d'être ultérieurement réutilisé.

De préférence, l'appareil de désinfection 1 comporte un dispositif de détection (non représenté) de la présence d'un élément à désinfecter dans l'enceinte de désinfection 10. De manière connue, le dispositif de détection peut comporter un capteur optique, par exemple une photodiode, adapté à mesurer une variation d'une caractéristique optique de l'enceinte de désinfection 10, la variation étant représentative de la présence ou non de l'élément à désinfecter. Le dispositif de détection comporte une unité de commande permettant d'activer et de désactiver le dispositif de nébulisation 20 et le dispositif d'injection 30.

Le fonctionnement de l'appareil de désinfection 1 est maintenant décrit, en référence aux figures 1 et 2.

On introduit dans l'enceinte de désinfection 10 un ou plusieurs éléments à désinfecter, par exemple une main d'un utilisateur. La main est introduite de manière à être placée de manière sensiblement colinéaire à l'axe longitudinal Δ.

Le détecteur de présence détecte la présence de la main à l'intérieur de l'enceinte de désinfection 10, et active le dispositif de nébulisation 20. Le nébuliseur piézoélectrique 24 provoque alors la nébulisation du liquide désinfectant dans la chambre de nébulisation 21. Le liquide désinfectant est alors transformé d'une phase liquide continue en un aérosol qui se forme dans la chambre de nébulisation 21.

Le dispositif d'injection 30 est activé, de manière simultanée ou légèrement différée vis-à-vis de l'activation du dispositif de nébulisation 20. Le ventilateur 36 provoque alors un écoulement d'air s'introduisant dans la chambre de nébulisation 21, ce qui conduit l'aérosol à s'écouler dans le conduit d'injection 34, et à être introduit dans l'enceinte de désinfection 10 au travers de l'orifice d'injection 33, ici sensiblement en direction de l'axe longitudinal Δ.

Par ailleurs, de l'air est aspiré par le ventilateur 36 et est injecté dans l'enceinte de désinfection 10 au travers de l'orifice d'injection 37, suivant la direction d'injection D₁ qui, en projection dans un plan perpendiculaire à l'axe longitudinal Δ, n'est pas sécante de l'axe longitudinal Δ, provoquant ainsi la formation d'un écoulement d'air, entraînant alors l'aérosol présent, de manière rotative autour de l'axe longitudinal Δ. Les gouttelettes de l'aérosol désinfectant suivent l'écoulement rotatif d'air autour de l'axe Δ, et donc autour de la main, et se déposent alors sur sensiblement toute la surface de la main. Le taux de recouvrement de la surface de la main par les gouttelettes de la solution désinfectante est ainsi particulièrement élevé, ce qui améliore le taux de désinfection de la main. La main reste libre de bouger dans l'enceinte de désinfection 10, améliorant ainsi le taux de recouvrement par l'aérosol désinfectant. Par ailleurs, le taux de recouvrement de la main par l'aérosol est élevé, sans qu'il soit nécessaire d'utiliser plusieurs injecteurs d'aérosol distribués autour de l'axe longitudinal Δ, et sans qu'il soit nécessaire de prévoir un écarteur obligeant l'utilisateur à écarter les doigts de la main.

Avantageusement, le ventilateur de sortie 39 est activé, ce qui provoque une convection forcée additionnelle dans l'enceinte de désinfection 10, allant de l'entrée 12 à la sortie 13. Ainsi, l'aérosol, tout en tournant autour de l'axe longitudinal Δ pour se déposer sur sensiblement toute la surface de la main, s'écoule en direction de la sortie 13, limitant ainsi la perte de l'aérosol par diffusion de celui-ci hors de l'enceinte 10 au travers de l'ouverture d'entrée 12 tout en améliorant l'homogénéité du dépôt de l'aérosol sur la surface de la main.

L'aérosol non utilisé est collecté par le filtre de condensation 41 et s'écoule dans le conduit 42 jusqu'au réservoir 44 de collecte. Les gouttelettes s'étant déposées sur la surface interne de la paroi 11 et ayant coalescé peuvent s'écouler jusqu'à l'orifice de collecte 43, pour s'écouler ensuite jusqu'au réservoir 44. Le liquide désinfectant non utilisé est ainsi récupéré pour ensuite, après traitement, être avantageusement réutilisé.

Après une durée donnée, définie par exemple par l'unité de commande ou par l'utilisateur, ce dernier retire sa main de l'enceinte de désinfection 10, et les dispositifs de nébulisation 20 et d'injection 30 peuvent être désactivés. Une phase préalable de séchage de la main par envoi d'un flux d'air chaud peut également être prévue.

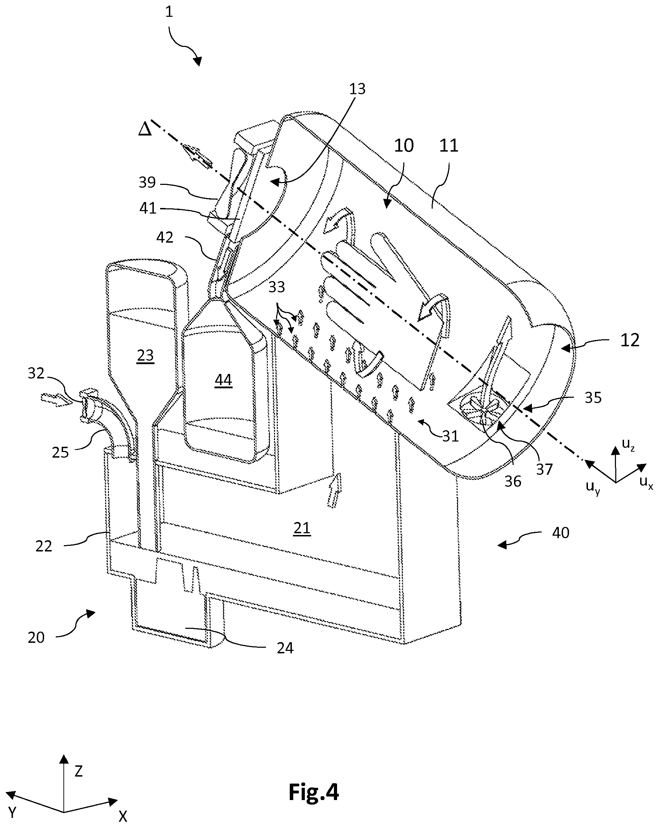

La figure 4 illustre de manière schématique un appareil de désinfection 1 selon une variante du premier mode de réalisation. L'appareil de désinfection 1 se distingue de celui illustré sur les figures 1 et 2 essentiellement en ce que la chambre de nébulisation 21 et l'enceinte de désinfection 10 ne sont plus délimitées par deux parois 11, 22 distinctes, mais sont ici séparées l'une de l'autre par une même paroi 11.

La paroi 11 de séparation comporte au moins un orifice d'injection 33 d'aérosol, et ici une pluralité d'orifices d'injection 33, traversant la paroi 11 de manière à permettre le passage de l'aérosol dans la chambre de nébulisation 21 directement dans l'enceinte de désinfection 10. La chambre de nébulisation 21 n'est ainsi plus reliée fluidiquement à l'enceinte de désinfection 10 par un conduit d'injection 34 d'aérosol formé par exemple d'un tuyau flexible ou rigide. On limite ainsi les éventuelles pertes de charge associées à de tels conduits, notamment les pertes de charge singulières formées par les changements brusques d'orientation des conduits susceptibles de perturber l'injection de l'aérosol dans l'enceinte de désinfection 10.

Ainsi, lors de l'activation du dispositif d'injection 30, le ventilateur 32 provoque un écoulement d'air entrant dans la chambre de nébulisation 21, ce qui induit l'introduction de l'aérosol dans l'enceinte de désinfection 10 au travers des orifices d'injection 33.

Par ailleurs, dans cet exemple, le réservoir de collecte 44 est situé du côté de la sortie 13 de l'enceinte de désinfection 10 et non plus du côté de l'entrée 12 de cette dernière, permettant ainsi d'améliorer la compacité de l'appareil de désinfection 1.

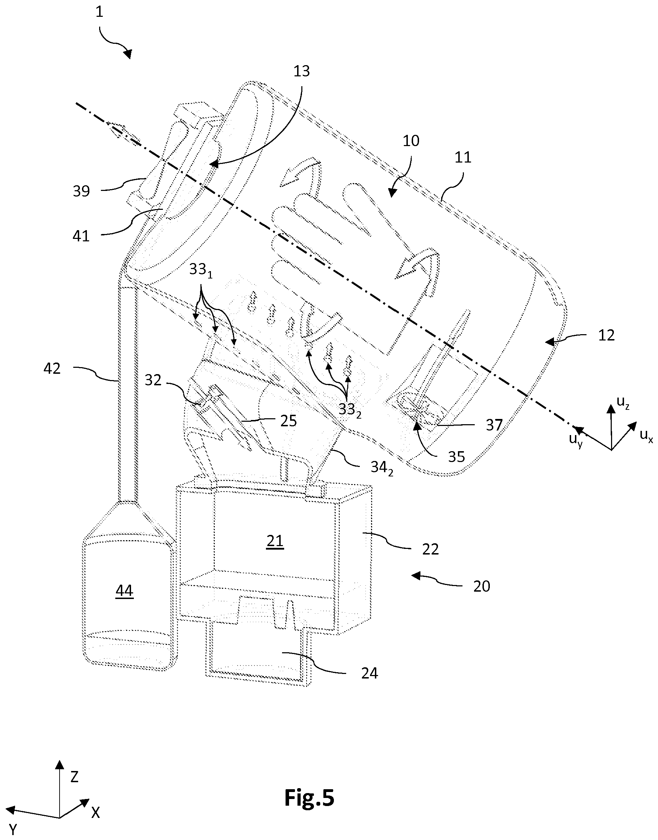

La figure 5 illustre une autre variante du dispositif de désinfection 1 selon le premier mode de réalisation. Ici, le dispositif d'injection comporte plusieurs ensembles d'orifices d'injection d'aérosol, ici deux ensembles 33₁, 33₂, chaque ensemble étant relié à la chambre de nébulisation 21 par un conduit 34₁, 34₂ distinct. Les deux ensembles 33₁ et 33₂ sont ici agencés de manière sensiblement symétrique par rapport à un plan passant par l'axe Δ. Les orifices 33 de chaque ensemble 33₁, 33₂ sont agencés suivant l'axe Δ. La fig.5 illustre plus particulièrement le conduit 34₂ reliant la chambre 21 à l'ensemble d'orifices 33₂. Dans cet exemple, le ventilateur 32 et le conduit d'entrée 25 peuvent être situés entre les deux conduits 34₁ et 34₂. Les autres éléments du dispositif de désinfection 1 sont identiques ou similaires à ceux déjà décrits et ne sont pas détaillés au nouveau. Cet agencement des ensembles d'orifices 33₁, 33₂ et leur liaison fluidique à la chambre 21 permettent notamment d'obtenir un débit plus important d'aérosol injecté dans l'enceinte de désinfection et/ou une injection plus homogène dans l'enceinte 10.

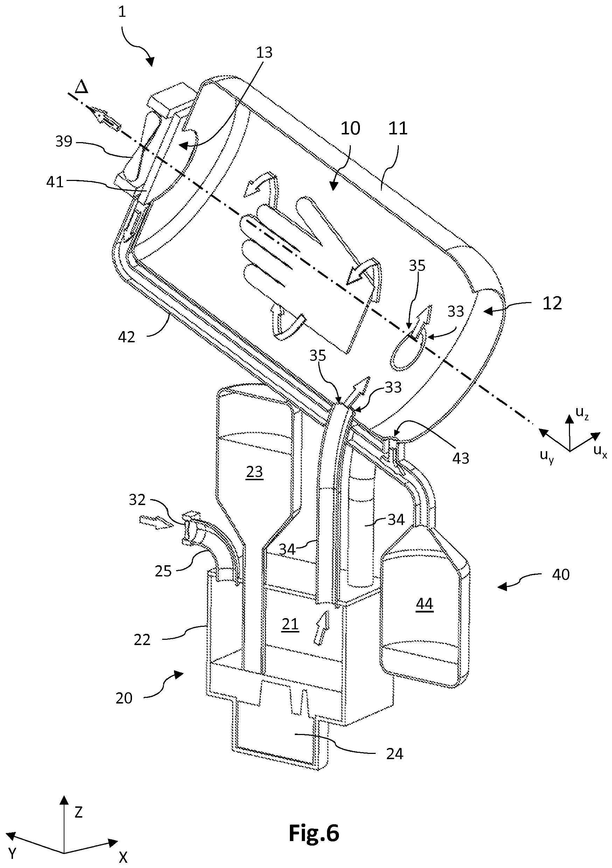

La figure 6 illustre de manière schématique un appareil de désinfection 1 selon une deuxième mode de réalisation. L'appareil de désinfection 1 se distingue de celui illustré sur les figures 1 et 2 essentiellement en ce que le dispositif d'injection 30 comporte au moins un injecteur d'aérosol 35 adapté à injecter également de l'air pour générer un flux d'aérosol rotatif autour de l'axe longitudinal Δ.

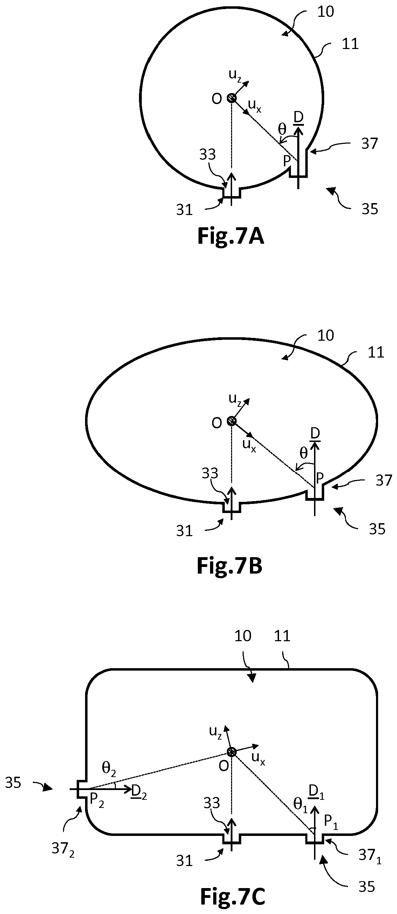

Dans cet exemple, comme l'illustre schématiquement la figure 7A, le dispositif d'injection 30 comporte, outre un injecteur d'aérosol 31 identique ou similaire à celui présent dans l'appareil de désinfection 1 selon le premier mode de réalisation, au moins un injecteur d'air 35 et d'aérosol ayant une direction d'inclinaison D inclinée, en projection dans un plan perpendiculaire à l'axe longitudinal Δ et vis-à-vis de l'axe de position OP correspondant, cet axe de position étant orthogonal à l'axe longitudinal Δ, et passant par ledit axe longitudinal Δ et l'orifice d'injection considéré. Cet injecteur d'air 35 et d'aérosol est formé d'un conduit 34 reliant la chambre de nébulisation 21 et débouchant dans l'enceinte de désinfection 10 par l'orifice d'injection 33. Le conduit d'injection 34 est orienté, au niveau de l'orifice d'injection 33, de sorte que son axe longitudinal coïncide avec la direction d'injection désirée. Ainsi, lors de l'activation du dispositif d'injection 30, le ventilateur 32 provoque un écoulement d'air entrant dans la chambre de nébulisation 21, ce qui induit l'introduction de l'aérosol dans l'enceinte de désinfection 10 par l'injecteur d'aérosol 31 d'une part, et par l'injecteur d'air 35 et d'aérosol d'autre part. Le flux rotatif est alors obtenu dans l'enceinte de désinfection 10.

Les figures 7B et 7C illustrent des variantes de l'appareil de désinfection 1 selon le deuxième mode de réalisation, qui se distinguent de l'appareil représenté sur la figure 7A notamment en ce que le dispositif d'injection 30 comporte plusieurs injecteurs 35 d'air et d'aérosol. Dans ce cas, les angles d'inclinaison des injecteurs 35 d'air et d'aérosol présentent avantageusement le même signe, dans le plan (uₓ,u_{z}) perpendiculaire à l'axe longitudinal Δ, de manière à renforcer le caractère rotatif du flux d'air, et donc d'aérosol, autour de l'axe longitudinal Δ. Elles illustrent schématiquement une enceinte de désinfection 10 de section droite, respectivement ovale et rectangulaire à coins arrondis, dans un plan de coupe (uₓ,u_{z}) orthogonal à l'axe longitudinal Δ. Le dispositif d'injection 30 comporte un seul injecteur d'aérosol 31 et deux injecteurs 35 d'air et d'aérosol.

L'injecteur d'aérosol 31 ne participe pas à générer un flux d'air rotatif autour de l'axe longitudinal Δ dans la mesure où sa direction d'injection est sensiblement sécante de l'axe Δ. Les deux injecteurs 35 d'aérosol et d'air, non seulement introduisent l'aérosol dans l'enceinte 10, mais génèrent un flux d'air rotatif dans la mesure où leur direction d'injection est inclinée, en projection dans un plan perpendiculaire à l'axe Δ et vis-à-vis de leur axe de position propre, et dans la mesure où les angles d'inclinaison sont de même signe.

Ainsi, un premier injecteur 35₁ d'air et d'aérosol présente une direction d'injection D₁ qui forme un angle d'inclinaison θ₁ par rapport à l'axe de position porté par le vecteur P₁O, ici de l'ordre de +40° environ. Le deuxième injecteur 35₂ d'air et d'aérosol présente une direction d'injection D₂ qui forme un angle d'inclinaison θ₂ par rapport à l'axe de position correspondant porté par le vecteur P₂O, ici également de l'ordre de +30°. Les angles d'inclinaison sont de même signe, de sorte que la convection forcée dans l'enceinte de désinfection 10 est bien rotative autour de l'axe longitudinal Δ.

Des modes de réalisation particuliers viennent d'être décrits. Différentes variantes et modifications apparaîtront à l'homme du métier. Ainsi, l'élément à désinfecter peut être d'autres membres d'un utilisateur, par exemple le pied, voire être un objet introduit et maintenu par l'utilisateur dans l'enceinte de désinfection 10.

## Revendications

1. Appareil de désinfection (1), comportant :
o une enceinte de désinfection (10), délimitée par une paroi (11) comportant une ouverture d'entrée (12) par laquelle un élément à désinfecter peut être introduit, et une ouverture de sortie (13) opposée à l'ouverture d'entrée (12) suivant un axe longitudinal (Δ) suivant lequel s'étend longitudinalement la paroi (11) ;
o un dispositif de nébulisation (20), adapté à nébuliser un produit désinfectant sous forme d'aérosol ;
o un dispositif d'injection (30), comportant :
• au moins un injecteur d'aérosol (31), adapté à injecter dans l'enceinte de désinfection (10) l'aérosol formé par le dispositif de nébulisation (20) ;
**caractérisé en ce que** le dispositif d'injection (30) comporte en outre :
• au moins un injecteur d'air (35), adapté à injecter de l'air au travers d'un orifice d'injection (37) de la paroi (11), suivant une direction d'injection (D), et à générer une convection forcée dans l'enceinte de désinfection (10) et rotative autour de l'axe longitudinal (Δ), l'injecteur d'air (35) étant agencé de sorte que la direction d'injection (D) est, en projection dans un plan perpendiculaire à l'axe longitudinal (Δ), inclinée vis-à-vis d'un axe dit de position (PO) ledit axe de position (PO) étant perpendiculaire à l'axe longitudinal (Δ) et passant par ledit orifice d'injection (37) dudit injecteur d'air (35).

2. Appareil de désinfection (1) selon la revendication 1, dans lequel le dispositif d'injection (30) comporte en outre un moyen de convection forcée (39), agencé vis-à-vis de l'enceinte de désinfection (10) de manière à générer un écoulement d'air allant de l'ouverture d'entrée (12) à l'ouverture de sortie (13).

3. Appareil de désinfection (1) selon la revendication 1 ou 2, dans lequel le dispositif d'injection (30) comporte au moins un injecteur d'air (35), et au moins un injecteur d'aérosol (31) distinct de l'injecteur d'air (35).

4. Appareil de désinfection (1) selon la revendication 1 ou 2, dans lequel le dispositif d'injection (30) comporte au moins un injecteur (35) d'air et d'aérosol adapté à former ledit injecteur d'air (35) et ledit injecteur d'aérosol (31).

5. Appareil de désinfection (1) selon l'une quelconque des revendications 1 à 4, comportant une pluralité d'injecteurs d'air (35) présentant chacun, en projection dans le plan perpendiculaire à l'axe longitudinal (Δ), un angle d'inclinaison (θ) non nul formé par sa direction d'injection (D) vis-à-vis de son axe de position (PO), les angles d'inclinaison étant de même signe.

6. Appareil de désinfection (1) selon l'une quelconque des revendications 1 à 5, dans lequel un filtre (41) apte à collecter l'aérosol est situé en regard de l'ouverture de sortie (13) de l'enceinte de désinfection (10).

7. Appareil de désinfection (1) selon l'une quelconque des revendications 1 à 6, dans lequel l'enceinte de désinfection (10) est agencée de sorte que l'axe longitudinal (Δ) est incliné vis-à-vis de l'axe de la gravité, l'ouverture de sortie (13) étant située au-dessus de l'ouverture d'entrée (12).

8. Appareil de désinfection (1) selon l'une quelconque des revendications 1 à 7, dans lequel l'injecteur d'air (35) comporte un ventilateur (36).

9. Appareil de désinfection (1) selon l'une quelconque des revendications 1 à 8, dans lequel le dispositif de nébulisation (20) comporte une chambre de nébulisation (21) comportant une solution liquide désinfectante, dans laquelle est situé un nébuliseur (24) adapté à transformer la solution liquide désinfectante en aérosol, l'injecteur d'aérosol (31) comportant au moins un conduit d'injection (34) reliant fluidiquement la chambre de nébulisation (21) à l'enceinte de désinfection (10).

10. Appareil de désinfection (1) selon l'une quelconque des revendications 1 à 8, dans lequel le dispositif de nébulisation (20) comporte une chambre de nébulisation (21) comportant une solution liquide désinfectante, dans laquelle est situé un nébuliseur (24) adapté à transformer la solution liquide désinfectante en aérosol, la chambre de nébulisation (21) et l'enceinte de désinfection (10) étant chacune délimitée au moins en partie par une même paroi (11) comportant au moins un orifice d'injection (33) traversant autorisant le passage de l'aérosol de la chambre de nébulisation (21) dans l'enceinte de désinfection (10).

## Patentansprüche

1. Desinfektionsvorrichtung (1) aufweisend:
o einen Desinfektionsraum (10), der von einer Wand (11) mit einer Einlassöffnung (12), durch die ein zu desinfizierendes Element eingeführt werden kann, und einer Auslassöffnung (13) gegenüber der Einlassöffnung (12) entlang einer Längsachse (Δ) begrenzt wird, entlang welcher sich die Wand (11) in Längsrichtung erstreckt;
o eine Zerstäubungsvorrichtung (20), die geeignet ist, ein aerosolförmiges Desinfektionsmittel zu zerstäuben;
o eine Einspritzvorrichtung (30), aufweisend:
• wenigstens einen Aerosoleinspritzer (31), der geeignet ist, in den Desinfektionsraum (10) das Aerosol einzuspritzen, das von der Zerstäubungsvorrichtung (20) gebildet wird;
**dadurch gekennzeichnet, dass** die Einspritzvorrichtung (30) ferner Folgendes aufweist:
• wenigstens einen Lufteinspritzer (35), der geeignet ist, Luft durch eine Einspritzöffnung (37) der Wand (11) in einer Einspritzrichtung (D) einzuspritzen und eine erzwungene Konvektion im Desinfektionsraum (10) und um die Längsachse (Δ) drehend zu erzeugen, wobei der Lufteinspritzer (35) so angeordnet ist, dass die Einspritzrichtung (D) in Projektion in einer Ebene senkrecht zur Längsachse (Δ) bezogen auf eine Positionsachse (PO) geneigt ist, wobei die Positionsachse (PO) senkrecht zur Längsachse (Δ) und durch die Einspritzöffnung (37) des Lufteinspritzers (35) verläuft.

2. Desinfektionsvorrichtung (1) nach Anspruch 1, wobei die Einspritzvorrichtung (30) ferner ein Mittel für erzwungene Konvektion (39) aufweist, das gegenüber dem Desinfektionsraum (10) so angeordnet ist, dass eine Luftströmung von der Einlassöffnung (12) zur Auslassöffnung (13) erzeugt wird.

3. Desinfektionsvorrichtung (1) nach Anspruch 1 oder 2, wobei die Einspritzvorrichtung (30) wenigstens einen Lufteinspritzer (35) und wenigstens einen Aerosoleinspritzer (31), der vom Lufteinspritzer (35) gesondert ist, aufweist.

4. Desinfektionsvorrichtung (1) nach Anspruch 1 oder 2, wobei die Einspritzvorrichtung (30) wenigstens einen Luft- und Aerosoleinspritzer (35) aufweist, der geeignet ist, den Lufteinspritzer (35) und den Aerosoleinspritzer (31) zu bilden.

5. Desinfektionsvorrichtung (1) nach einem der Ansprüche 1 bis 4, aufweisend eine Mehrzahl von Lufteinspritzern (35), die jeweils in Projektion in der Ebene senkrecht zur Längsachse (Δ) einen Neigungswinkel (θ) ungleich null aufweisen, der von seiner Einspritzrichtung (D) bezogen auf seine Positionsachse (PO) gebildet wird, wobei die Neigungswinkel vorzeichengleich sind.

6. Desinfektionsvorrichtung (1) nach einem der Ansprüche 1 bis 5, wobei sich ein Filter (41), der geeignet ist, das Aerosol aufzufangen, gegenüber der Auslassöffnung (13) des Desinfektionsraums (10) befindet.

7. Desinfektionsvorrichtung (1) nach einem der Ansprüche 1 bis 6, wobei der Desinfektionsraum (10) so angeordnet ist, dass die Längsachse (Δ) bezogen auf die Achse der Schwerkraft geneigt ist, wobei sich die Auslassöffnung (13) über der Einlassöffnung (12) befindet.

8. Desinfektionsvorrichtung (1) nach einem der Ansprüche 1 bis 7, wobei der Lufteinspritzer (35) einen Ventilator (36) aufweist.

9. Desinfektionsvorrichtung (1) nach einem der Ansprüche 1 bis 8, wobei die Zerstäubungsvorrichtung (20) eine Zerstäubungskammer (21) mit einer flüssigen Desinfektionslösung aufweist, in der sich ein Zerstäuber (24) befindet, der geeignet ist, die flüssige Desinfektionslösung zu Aerosol zu zerstäuben, wobei der Aerosoleinspritzer (31) wenigstens eine Einspritzleitung (34) aufweist, die die Zerstäubungskammer (21) fluidisch mit dem Desinfektionsraum (10) verbindet.

10. Desinfektionsvorrichtung (1) nach einem der Ansprüche 1 bis 8, wobei die Zerstäubungsvorrichtung (20) eine Zerstäubungskammer (21) mit einer flüssigen Desinfektionslösung aufweist, in der sich ein Zerstäuber (24) befindet, der geeignet ist, die flüssige Desinfektionslösung zu Aerosol zu zerstäuben, wobei die Zerstäubungskammer (21) und der Desinfektionsraum (10) jeweils wenigstens teilweise von ein und derselben Wand (11) begrenzt sind, die wenigstens eine durchgehende Einspritzöffnung (33) aufweist, die den Durchtritt des Aerosols von der Zerstäubungskammer (21) in den Desinfektionsraum (10) ermöglicht.

## Claims

1. Disinfection appliance (1) comprising:
o a disinfection enclosure (10), bounded by a wall (11) comprising an inlet opening (12) via which an element that is to be disinfected can be introduced, and an outlet opening (13), opposite the inlet opening (12) along a longitudinal axis (Δ) along which the wall (11) extends longitudinally;
o a nebulisation device (20) designed to nebulise a disinfectant product into the form of an aerosol;
o an injection device (30), comprising:
• at least one aerosol injector (31), designed to inject into the disinfection enclosure (10) the aerosol formed by the nebulisation device (20);
**characterized in that** the injection device (30) further comprises:
• at least one air injector (35), designed to inject air through at least one injection orifice (27) in the wall (11), in an injection direction (D), and to generate in the disinfection chamber (10) forced convection that also rotates about the longitudinal axis (Δ), the air injector (35) being arranged in such a way that the injection direction (D) is, in projection in a plane perpendicular to the longitudinal axis (Δ), inclined with respect to an axis referred to as the position axis (PO), said position axis (PO) being perpendicular to the longitudinal axis (Δ) and passing through said injection orifice (37) of said air injector (35).

2. Disinfection appliance (1) according to Claim 1, wherein the injection device (30) further comprises a forced-convection means (39) arranged with respect to the disinfection enclosure (10) in such a way as to generate an air flow extending from the inlet opening (12) to the outlet opening (13).

3. Disinfection appliance (1) according to Claim 1 or 2, wherein the injection device (30) comprises at least one air injector (35) and at least one aerosol injector (31) distinct from the air injector (35).

4. Disinfection appliance (1) according to Claim 1 or 2, wherein the injection device (30) comprises at least one air and aerosol injector (35) designed to form said air injector (35) and said aerosol injector (31).

5. Disinfection appliance (1) according to any one of Claims 1 to 4, comprising a plurality of air injectors (35) each having, in projection in the plane perpendicular to the longitudinal axis (Δ), a non-zero angle of inclination (θ) formed by its injection direction (D) with respect to its position axis (PO), the angles of inclination being of the same sign.

6. Disinfection appliance (1) according to any one of Claims 1 to 5, wherein a filter (41) able to collect the aerosol is situated facing the outlet opening (13) of the disinfection enclosure (10).

7. Disinfection appliance (1) according to any one of Claims 1 to 6, wherein the disinfection enclosure (10) is arranged in such a way that the longitudinal axis (Δ) is inclined with respect to the axis of gravity, the outlet opening (13) being situated above the inlet opening (12).

8. Disinfection appliance (1) according to any one of Claims 1 to 7, wherein the air injector (35) comprises a fan (36).

9. Disinfection appliance (1) according to any one of Claims 1 to 8, wherein the nebulisation device (20) comprises a nebulisation chamber (21) containing a disinfectant liquid solution, in which chamber there is a nebuliser (24) designed to convert the disinfectant liquid solution into an aerosol, the aerosol injector (31) comprising at least one injection duct (34) for fluidically connecting the nebulisation chamber (21) to the disinfection enclosure (10).

10. Disinfection appliance (1) according to any one of Claims 1 to 8, wherein the nebulisation device (20) comprises a nebulisation chamber (21) containing a disinfectant liquid solution, in which chamber there is a nebuliser (24) designed to convert the disinfectant liquid solution into an aerosol, the nebulisation chamber (21) and the disinfection enclosure (10) each being bounded at least in part by the one same wall (11) comprising at least one injection through-orifice (33) allowing aerosol to pass from the nebulisation chamber (21) into the disinfection enclosure (10).
